# EUROPEAN PATENT SPECIFICATION

(11) **EP 0 863 782 B1**
(45) Date of publication and mention of the grant of the patent: **16.08.2001**
(21) Application number: 96940098.5
(22) Date of filing: 02.12.1996
(51) Int. Cl.: B01D 15/08, C07C 231/24, C07C 237/46

(54) **REVERSED PHASE CHROMATOGRAPHIC PROCESS**
VERFAHREN ZUR UMKEHRPHASEN-CHROMATOGRAPHIE
PROCEDE DE CHROMATOGRAPHIE EN PHASES INVERSEES

(30) Priority: 01.12.1995 IE 950904
(43) Date of publication of application: 16.09.1998
(73) Proprietor: Mallinckrodt Medical Imaging-Ireland, Dublin 2 (IE)
(72) Inventor: COLLINS, Elizabeth, Dublin 18 (IE); MULDOON, Stephen, Dunboyne, County Meath (IE); O'CALLAGHAN, Brendan, Rathoath, County Meath (IE); CAROLAN, Dominic, Malahide, County Dublin (IE)
(74) Representative: O'Brien, John Augustine
(86) International application number: IE9600076
(87) International publication number: WO9720609

(56) References cited:
- WO-A-91/12868
- GB-A- 2 287 024
- JOURNAL OF CHROMATOGRAPHY, vol. 366, 1986, AMSTERDAM, pages 299-309, XP000605567 W. SKJOLD AND A. BERG: "PREPARATIVE LIQUID CHROMATOGRAPHY IN THE FIELD OF X-RAY CONTRAST AGENTS"

## Description

### Background of the Invention

This invention relates to a process for the reversed phase chromatographic decolorisation, separation and purification of water-soluble nonionic contrast media compounds from solutions containing nonionic compound impurities.

A process of this type is described in WO 91/12868A. While this process is very successful for maximum manufacturing efficiency, there is a need to optimise the yield of the nonionic contrast media compound.

### Summary of the Invention

The object of the invention, therefore, is to provide an improved factory scale process for the decolorization, reversed phase chromatographic separation and purification of water-soluble, nonionic contrast media compounds in which the yield of the desired contrast media compound is maximised.

According to the invention there is provided a process for the reversed phase chromatographic decolorisation, separation and purification of water-soluble nonionic contrast media compounds from solutions containing nonionic compound impurities comprising the steps of:-
passing a solution containing a water-soluble, nonionic contrast media compound and nonionic compounds as impurities through a chromatographic packing material in a chromatographic column; and
eluting said column to produce primary eluate containing substantially pure, water-soluble, nonionic contrast media compound;
passing a solvent through said column to wash the chromatographic packing material and form a mixture comprising solvent, nonionic contrast media compound and, as impurities, nonionic compounds;
distilling said mixture to provide a distillate of substantially pure solvent and a bottoms product comprising the aqueous solution containing the nonionic contrast media compound and nonionic compounds as impurities;
concentrating the bottoms product;
crystallising said impurities;
filtering said bottoms product containing impurities to remove the crystallised impurities as a filter cake and to provide a filtrate containing the nonionic contrast media compound; and
passing the filtrate through a chromatographic column to form a tertiary eluate containing nonionic contrast media compound.

Typically, said tertiary eluate is concentrated by evaporation to form a concentrated tertiary eluate. Preferably, the tertiary eluate is concentrated under vacuum in order to minimise any potential for thermal degradation.

In a preferred embodiment of the invention, said concentrated tertiary eluate is mixed with primary and/or secondary eluate from the main process and the combined eluate is purified and dried to produce a nonionic contrast media compound.

Preferably, the solvent is a lower alkanol, especially methanol.

The novel process of the present invention is applicable to the general decolorisation and separation of nonionic compound impurities from water-soluble, nonionic contrast media compounds, nonionic nuclear diagnostic imaging compounds or alternatively, MRI agents. The nonionic contract media compounds include x-ray contract media compounds such as N,N'-bis(2,3-dihydroxypropyl)-5-[N-(2-hydroxyethyl)glycolamido]-2,4,6-triiodo-isophthalamide(ioversol), N,N'-bis(2,3-dihydroxypropyl)-5-[N-(2,3-dihydroxypropyl)acetamido]-2,4,6-triiodoisophthalamide(iohexol), N,N'-bis(1,3-dihydroxypropyl)-5-lactylamido-2,4,6-triiodoisophthalamide(iopamidol), ), 2[3-acetamido-2,4,6-triiodo-5- (N-methylacetamido) benzamido]-2-deoxy-D-glucose(metrazamide), N,N'-bis(2,3-hydroxy-propyl)-2,4,6-triiodo-5-(2-keto-L-gulonamido) isophthalimide(iogulamide), 5,5 '-[malonylbis[(2-hydroxy-ethyl)imino]]bis[N,N'-bis[2-hydroxy-1-(hydroxymethylethyl]-2,4,6-triiodoisophthalamide(iodecimol)5,5'-[(2-hydroxytrimethylene)bis(acetylimino)]bis[N,N'-bis(2,3-dihydroxypropyl)-2,4,6-triiodoisophthalamide(iodixanol), 3-[N-(2-hydroxyethyl)acetamido]-2,4,6-triiodo-5-(methylcarbamoyl)-D-glucoanilide(ioglucol), N,N'-bis(2,3-dihydroxypropyl)-2,4,6-triiodo-5-(N-methylglycolamido) isophthalamide(iomeprol), N,N'-bis(2,3-dihydroxypropyl)-5-[N-(2-hydroxy-3-methoxypropyl)acetamido]-2,4,6-triiodoisophthalamide(iopentol), N,N'-bis(2,3-dihydroxypropyl)2,4,6-triiodo-5-(2-methoxyacetamido)-N-methylisophthal-amide(iopromide), 3,5-diacetamido-2,4,6-triiodo-N-methyl-N[[methyl(D-gluco2,3,4-5,6-pentahydroxyhexyl)carbamoyl]methyl]benzamide(iosarcol), N,N,N',N',N",N"-hexakis(2-hydroxyethyl)-2,4,6-triiodo-1,3,5-benzenetricarboxamide(iosimide, 5,5'[thiobis(ethylenecarbonylimino)]bis[N,N-bis(2,3-dihydroxypropyl)-2,4,6-triiodo-N,N'-dimethylisophthalamide (iotasul), and 5,5'-[malonylbis(methylimino)]bis[N,N'-bis[2,3-dihydroxy-1(hydroxy-methyl)propyl]-2,4,6-triiodoisophthalamide (iotrolan).

As used herein, the term "nonionic contrast media compounds" includes, and the present invention is applicable to, nonionic magnetic resonance imaging (MRI) agent ligands and neutral (or nonionic) metal complexes of their ligands with suitable metals from the first, second, or third row transition elements or the lanthanide or actinide series. Typical ligands include N,N"-bis[N-(2,3-dihydroxypropyl)carbamoylmethyl]diethylenetriamine-N,N',N"-triacetic acid, N,N'-bis[N-(2,3-dihydroxypropyl) carbamoylmethyl]ethylene-diamine-N,N'-diacetic acid, N,N'"-bis[N-(2-hydroxyethyl)carbamoylmethyl] triethylenetetraamine-N,N',N",N' "-tetraacetic acid, N,N"-bis[N-(1-hydroxymethyl-2,3-dihydroxypropyl) car-bamoylmethyl]diethylenetriamine-N,N',N"-triaacetic acid and N,N'-bis[N-(2-hydroxyethyl)carbamoylmethyl] ethylenediamine-N,N'-diacetic acid. Examples of various other complexes, nonionic contrast media compounds and MRI agents which may be purified through the process of the invention are known to those skilled in the art.

In one particular embodiment of the invention, the contrast media compound is the nonionic X-ray contrast media compound N,N'-bis(2,3-dihydroxypropyl)-5-[N-(2-hydroxyethyl)glycolamido]-2,4,6-triiodoisophthalamide (Ioversol) described in US 4,396,598.

### Description of the Drawings

The invention will be more clearly understood from the following description thereof, given by way of example only, in which:-
Fig. 1 is a schematic flow diagram of an improved recovery process according to the invention; and
Fig. 2 is a flowchart of some details of the process.

### Description of Preferred Embodiment

It has been found that the process described in WO 91/12868A may be further improved by extracting the relatively small but significant amount of nonionic contrast medium compound remaining on the column packing and recovering the nonionic contrast medium compound.

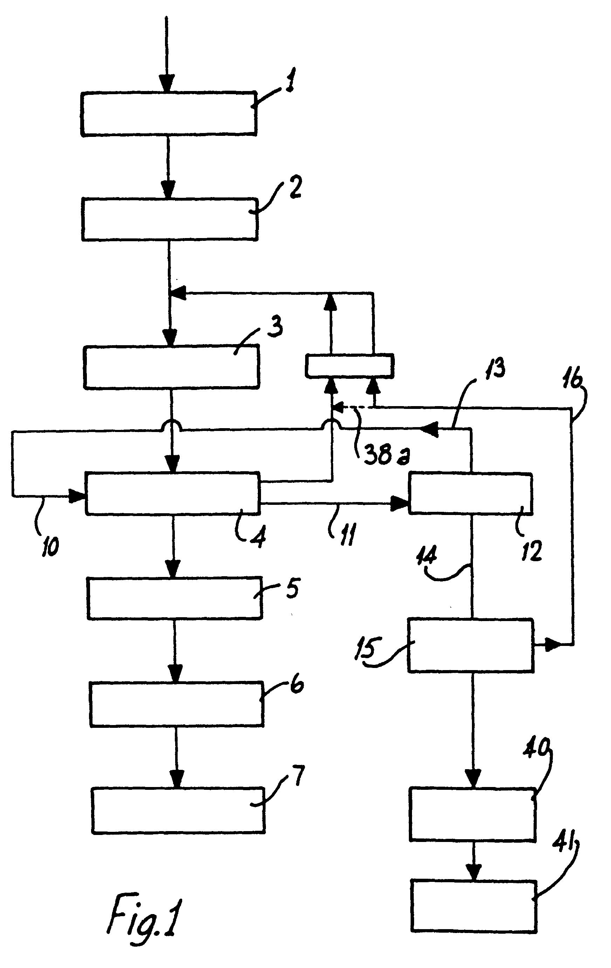

In the improved factory scale process of the invention illustrated in Fig. 1, the first step is decolorisation 1 followed by deionisation 2 and evaporation 3. After evaporation, the nonionic contrast medium compound is purified in step 4, concentrated by evaporation in step 5 and dried in step 6 to provide a finished nonionic contrast medium product 7.

In the purification process, methanol delivered along line 10 is used to wash the chromatographic purification columns and the mixture containing methanol and nonionic contrast media compound from the column packing is delivered along a line 11 to a solvent recovery still 12. The substantially pure methanol distillate from the recovery still 12 is recycled along line 13 to the main methanol intake 10 and bottoms product 14 from the methanol recovery still 12 is delivered to a nonionic contrast medium recovery process 15 which is described in more detail below. Waste material from the recovery process may be deiodinated at 40 prior to waste treatment 41. Nonionic contrast medium recovered in the recovery process 15 is recycled along line 16 for combining with a primary and/or secondary eluate stream either after deionisation as illustrated or before deionisation.

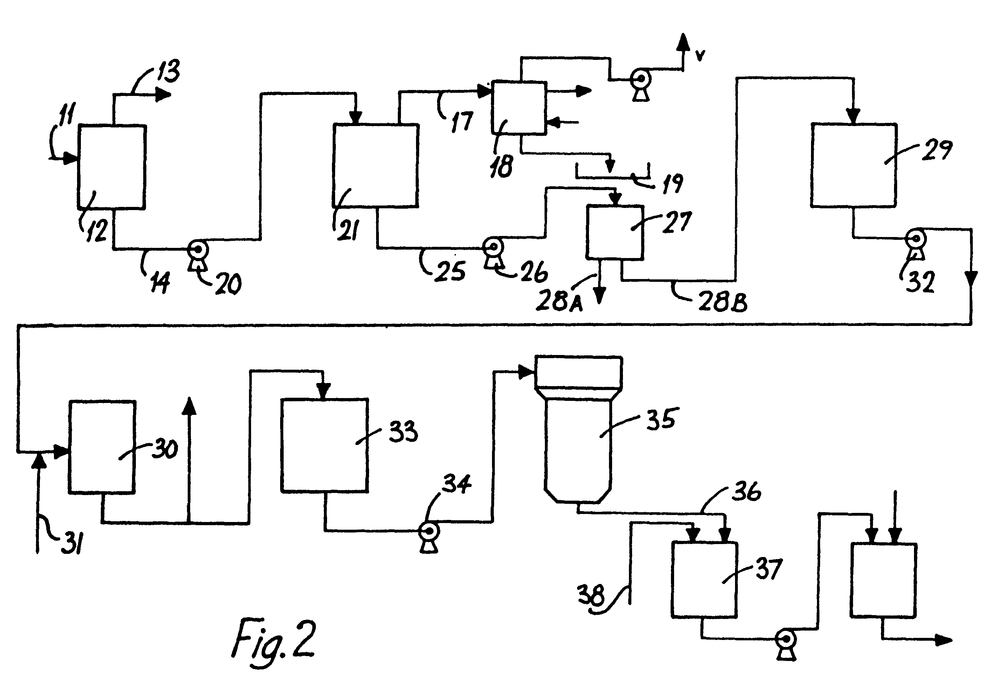

The bottoms product 14 is an aqueous solution containing the nonionic contrast media compound and nonionic compounds as impurities. In more detail, and referring to Fig. 2, the bottoms product 14 from the methanol still 12 is pumped by a pump 20 to a vessel 21 in which the bottoms are concentrated and impurities are crystallised. Tops product 17 from the still 12 is condensed in a condenser 18, the condensate being collected in a receiver 19 and gases being discharged to vent V.

A stream 25 containing concentrated bottoms product and crystallised impurities is then pumped to a filter 27 in which the solids impurities are filtered out in the form of a filter cake 28A, with the filtrate 28B containing the nonionic contrast medium passing into a holding tank 29.

From the holding tank 29, the filtrate is delivered by a pump 32 for purification by reverse phase chromatography in a chromatographic column 30 using the techniques described in WO 91/12868A.

Tertiary eluent from the column 30 is delivered into a holding tank 33 and then pumped by a pump 34 to an evaporator 35 in which the tertiary eluent is concentrated under vacuum to form a concentrate 36 which may be combined in a tank 37 with primary and secondary eluent 38 from the primary process described above. Alternatively, as illustrated by the interrupted line 38a in Fig. 1, the tertiary eluate may be combined with the secondary eluent prior to evaporation 35.

We have found that using the improved process of the invention has increased the yield of nonionic contrast medium by up to 3% per batch resulting in very significant improvements in productivity while minimising waste.

### EXAMPLE 1

The process outlined in Example 3 of WO 91/12868 was carried out. When the last purification column regeneration operation from the process described in WO 91/12868 was completed, the methanol recovery operation was continued until the methanol levels in the still bottoms were <3% w/v. The still bottoms solution was then tested for density, transferred to a 630 lt glass lined reactor, agitated and maintained at 40°C.

The reactor was then placed under a vacuum of ca. 0.15 barA and the temperature of the reactor contents were then brought up slowly to the solution boiling point of ca. 55-70°C. As water was evaporated from the solution, the solubility of impurity A decreased resulting in precipitation or crystallisation of the impurity. This concentration/crystallisation step was continued until the final required concentration of ca. 50-70% w/w was achieved, as estimated from the solution density.

Once the correct concentration was achieved, the reactor contents were cooled to <40°C. At this temperature vacuum was removed and cooling continued until the temperature was <20°C.

The contents of the reactor were then agitated for 1 hour to enhance the crystallisation of impurity A. After this holding period the contents were pumped through a horizontal plate filter and the filtrate was collected in a hold tank. Impurity A, crystallised in the glass lined reactor, was removed on the filter plates resulting in a clear filtrate, with a much reduced impurity level passing to the next step. The filter cake was washed and the wash recycled to the glass lined concentration/crystallisation vessel for addition to the next batch. The filter was then blown dry with compressed air and the filter split to remove the filter cake. The plates were then cleaned and the filter unit made ready for the next batch.

The filtrate from the above step was then purified by reverse phase chromatographic separation in a stainless steel column (610mm internal diameter x 996mm length) packed with approximately 155kgs of dry silanized chromatographic packing material, consisting of octadecylsilane bonded to solid silica particles ('ODS-Si'). The column bed was prepared by filling the column initially with ca. 150kgs of dry packing, while vibrating the column to ensure a uniform column bed. Approximately 790 lts of Methanol were then pumped through the column, followed by ca. 833 lts of Process Water. This process forces air from the packing material, compacting it to leave an empty space at the top of the column, which was then filled with additional fresh resin. The flushing procedure was then repeated and the column 'topped off' again with this process repeated until there was no further compression of the packing material. The column holds a total of ca. 155 kgs of packing material when completely packed.

The filtrate from the earlier filtration step was sampled for density and a calculation then performed using the volume of filtrate (based on tank level), the solution density and the purification loading ratio to determine the optimum number of runs required to effect separation of nonionic impurities A, B, C and D in the feed stream.

The required volume of solution, containing approximately 30 kgs of Ioversol and containing nonionic impurities A, B, C & D was pumped through a 0.2 micron filter onto the chromatographic column. The loading ratio of packaging material/total wt. nonionic compounds was approximately 7:1. Process water was then pumped through the column to elute the product.

The first ca. 300 lts of effluent, or eluate, from the chromatographic column was directed to drain. On detection of product (density at the outlet of the column exceeds 1.005 for > 3 seconds or a maximum of 300 lts eluate is reached) the eluate is then redirected to a holding tank for collection. This fraction, which will be termed the Ioversol 3rd Crop, to differentiate it from the 1st and 2nd cuts already generated in the standard process described in WO91/12868 contains ca. 85-95% of the purified Ioversol.

The chromatographic column was then flushed with a mixture of methyl alcohol and water which contained at least 50% methyl alcohol to remove non ionic compound impurities from the column and regenerate the column packing for reuse. The column was then reequilibrated with process water before reuse in purifying subsequent batches containing Ioversol and nonionic impurities A, B, C and D.

The 3rd crop chromatography fraction containing purified Ioversol was then concentrated on a single pass through a wiped film evaporator under a vacuum of ca. 0.28 barA to obtain a solution of ca. 3-6% concentration. This concentrated 3rd crop solution was then mixed with normal concentrated second cut material, with the composite material sampled to evaluate quality. On satisfactory review of the levels of nonionic impurities A, B, C and D in the 3rd crop and composite 3rd crop/2nd cut materials described above, the composite solution may then be recycled to a subsequent batch of crude, deionised Ioversol solution, for purification by reverse phase chromatographic separation as described in WO 91/12868.

This process illustrates that the combined 3rd crop and second fraction materials may be further reprocessed by recycling into the original column feed provided sufficient overall quality is maintained.

The process of the invention is applicable not only to Ioversol but also to the general decolorisation and separation of nonionic compound impurities from water-soluble, nonionic contrast media compounds in general, or MRI agents. Examples of such compounds and agents are given in WO 91/12868A.

The invention is not limited to the embodiments hereinbefore described which may be varied in detail.

## Claims

1. A process for the reversed phase chromatographic decolorisation (1), separation and purification (4) of water-soluble nonionic contrast media compounds (7) from solutions containing nonionic compound impurities comprising the steps of:-
passing a solution containing a water-soluble, nonionic contrast media compound and nonionic compounds as impurities through a chromatographic packing material in a chromatographic column (4);
eluting said column (4) to produce primary eluate containing substantially pure, water-soluble, nonionic contrast media compound;
passing a solvent through said column (4) to wash the chromatographic packing material and form a mixture comprising solvent, nonionic contrast media compound and, as impurities, nonionic compounds;
distilling said mixture to provide a distillate of substantially pure solvent and a bottoms product (14) comprising the aqueous solution containing the nonionic contrast media compound and nonionic compounds as impurities;
concentrating (21) the bottoms product (14);
crystallising said impurities (21);
filtering (27) said bottoms product (14) containing impurities to remove the crystallised impurities as a filter cake (28A) and to provide a filtrate (28B) containing the nonionic contrast media compound; and
passing the filtrate (28B) through a chromatographic column (30) to form a tertiary eluate containing nonionic contrast media compound.

2. A process as claimed in claim 1 wherein said tertiary eluate is concentrated by evaporation (35) to form a concentrated tertiary eluate.

3. A process as claimed in claim 2 wherein said tertiary eluate is concentrated under vacuum.

4. A process as claimed in claim 2 or 3 wherein said concentrated eluate is mixed with primary and/or secondary eluate (38) and the combined eluate (37) is purified and dried to produce a nonionic contrast media compound (7).

5. A process as claimed in any preceding claim wherein the solvent is a lower alkanol, preferably methanol.

6. A process as claimed in any preceding claim wherein the contrast media compound is the nonionic X-ray contrast media compound N,N'-bis(2,3-dihydroxypropyl)-5-[N-(2-hydroxyethyl)glycolamido]-2,4,6-triiodoisophthalamide.

## Patentansprüche

1. Verfahren für die Umkehrphasen-chromatographische Entfärbung (1), Abtrennung und Reinigung (4) von wasserlöslichen, nichtionischen Kontrastmittelverbindungen (7) aus Lösungen, die Verunreinigungen von nichtionischen Verbindungen enthalten, das die Schritte umfaßt:
Leiten einer Lösung, die eine wasserlösliche, nichtionische Kontrastmittelverbindung und nichtionische Verbindungen als Verunreinigungen enthält, durch ein chromatographisches Packmaterial in einer Chromatographiesäule (4);
Eluieren der Säule (4), um ein Primäreluat, das eine im wesentlichen reine, wasserlösliche, nichtionische Kontrastmittelverbindung enthält, zu erzeugen;
Leiten eines Lösungsmittels durch die Säule (4), um das chromatographische Packmaterial zu waschen und um eine Mischung, die Lösungsmittel, nichtionische Kontrastmittelverbindung und als Verunreinigungen nichtionische Verbindungen enthält, zu bilden;
Destillieren der Mischung, um ein Destillat eines im wesentlichen reinen Lösungsmittels und ein Bodenprodukt (14), das die wässrige Lösung, die nichtionische Kontrastmittelverbindung und nichtionische Verbindungen als Verunreinigungen enthält, umfaßt, bereitzustellen;
Konzentrieren (21) des Bodenproduktes (14);
Kristallisieren der Verunreinigungen (21);
Filtrieren (27) des Bodenprodukts (14), das Verunreinigungen enthält, um die kristallisierten Verunreinigungen als Filterkuchen (28A) zu entfernen und um ein Filtrat (28B), das die nichtionische Kontrastmittelverbindung enthält, bereitzustellen; und
Leiten des Filtrats (28B) durch eine Chromatographiesäule (30), um ein Tertiäreluat, das eine nichtionische Kontrastmittelverbindung enthält, zu bilden.

2. Verfahren gemäß Anspruch 1, wobei das Tertiäreluat durch Verflüchtigung (35) konzentriert wird, um ein konzentriertes Tertiäreluat zu bilden.

3. Verfahren gemäß Anspruch 2, wobei das Tertiäreluat unter Vakuum konzentriert wird.

4. Verfahren gemäß Anspruch 2 oder 3, wobei das konzentrierte Eluat mit Primär- und/oder Sekundäreluat (38) gemischt wird und das vereinigte Eluat (37) gereinigt und getrocknet wird, um eine nichtionische Kontrastmittelverbindung (7) zu ergeben.

5. Verfahren gemäß einem der vorhergehenden Ansprüche, wobei das Lösungsmittel ein niederes Alkanol, bevorzugt Methanol, ist.

6. Verfahren gemäß einem der vorhergehenden Ansprüche, wobei die Kontrastmittelverbindung die nichtionische Kontrastmittelverbindung für Röntgenstrahlen N,N'-Bis(2,3-Dihydroxypropyl)-5-[N-(2-hydroxyethyl)glycolamido]-2,4,6-trijodisophthalamid ist.

## Revendications

1. Procédé pour la décoloration (1), la séparation et la purification (4) par chromatographie en phase inversée, de composés (7) non ioniques hydrosolubles pour milieux de contraste à partir de solutions contenant des impuretés du composé non ionique, comprenant les étapes consistant à :
faire passer une solution contenant un composé non ionique hydrosoluble pour milieux de contraste et des composés non ioniques en tant qu'impuretés à travers un matériau de charge chromatographique dans une colonne chromatographique (4) ;
éluer ladite colonne (4) pour produire un éluat primaire contenant un composé non ionique hydrosoluble pour milieux de contraste, essentiellement pur ;
faire passer un solvant à travers ladite colonne (4) pour laver le matériau de charge chromatographique et former un mélange comprenant le solvant, le composé non ionique pour milieux de contraste et, comme impuretés, des composés non ioniques ;
distiller ledit mélange pour fournir un distillat de solvant pratiquement pur et de produit de bas de colonne (14) comprenant la solution aqueuse contenant le composé non ionique pour milieux de contraste et les composés non ioniques comme impuretés ;
concentrer (21) le produit de bas de colonne (14) ;
cristalliser lesdites impuretés (21) ;
filtrer (27) ledit produit de bas de colonne (14) contenant des impuretés afin d'éliminer les impuretés cristallisées sous forme d'un gâteau de filtre (28A) et fournir un filtrat (28B) contenant le composé non ionique pour milieux de contraste ; et
faire passer le filtrat (28B) à travers une colonne chromatographique (30) pour former un éluat tertiaire contenant le composé non ionique pour milieux de contraste.

2. Procédé tel que revendiqué dans la revendication 1, dans lequel ledit éluat tertiaire est concentré par évaporation (35) pour former un éluat tertiaire concentré.

3. Procédé tel que revendiqué dans la revendication 2, dans lequel ledit éluat tertiaire est concentré sous vide.

4. Procédé tel que revendiqué dans la revendication 2 ou 3, dans lequel ledit éluat concentré est mélangé avec l'éluat primaire et/ou secondaire (38) et l'éluat combiné (37) est purifié et séché pour produire un composé (7) non ionique pour milieux de contraste.

5. Procédé tel que revendiqué dans l'une quelconque des revendications précédentes, dans lequel le solvant est un alcanol inférieur, de préférence le méthanol.

6. Procédé tel que revendiqué dans l'une quelconque des revendications précédentes, dans lequel le composé pour milieux de contraste est le composé non ionique pour milieux de contraste pour rayons-X N,N'-bis(2,3-dihydroxypropyl)-5-[N-(2-hydroxyéthyl)glycolamido]-2,4,6-triiodoisophtalamide.
